# EUROPÄISCHE PATENTSCHRIFT

(11) **EP 0 929 541 B1**
(45) Veröffentlichungstag und Bekanntmachung des Hinweises auf die Patenterteilung: **27.06.2001**
(21) Anmeldenummer: 97909303.6
(22) Anmeldetag: 22.09.1997
(51) Int. Cl.: C07D 307/88

(54) **VERFAHREN ZUR HERSTELLUNG VON PHTHALIDEN**
PROCESS FOR PREPARING PHTHALIDES
PROCEDE DE PRODUCTION DE PHTALIDES

(30) Priorität: 01.10.1996 DE 19640554
(43) Veröffentlichungstag der Anmeldung: 21.07.1999
(73) Patentinhaber: BASF AKTIENGESELLSCHAFT, 67056 Ludwigshafen (DE)
(72) Erfinder: MASSONNE, Klemens, D-67368 Westheim (DE); BECKER, Rainer, D-67098 Bad Dürkheim (DE); NEUHAUSER, Horst, D-67373 Dudenhofen (DE); ZIMMERMANN, Horst, D-68199 Mannheim (DE); REIF, Wolfgang, D-67227 Frankenthal (DE); CRAMERS, Peter, CH-4416 Bubendorf (CH); NARDIN, Daniel, CH-4557 Horriwil (CH)
(74) Vertreter: Fitzner, Uwe, Dr.
(86) Internationale Anmeldenummer: EP9705186
(87) Internationale Veröffentlichungsnummer: WO9814441

(56) Entgegenhaltungen:
- EP-A- 0 542 037
- US-A- 2 114 696
- HOUBEN-WEYL: "Methoden der organischen Chemie, 6/2, " 1963 , GEORG THIEME VERLAG , STUTTGART XP002051920 in der Anmeldung erwähnt siehe Tabelle 33 siehe Seite 731 - Seite 733

## Beschreibung

Die vorliegende Erfindung betrifft ein Verfahren zur Herstellung von Phthaliden durch katalytische Hydrierung von Phthalsäureanhydriden mit Hilfe von suspendierten Raney-Katalysatoren.

Aus DE-C-28 03 319 ist es bekannt, Phthalid durch katalytische Hydrierung von Phthalsäureanhydrid in der Gasphase mit Kupfer und Aluminium enthaltenden Katalysatoren durchzuführen. Dieses Verfahren erfordert jedoch einen erhöhten Aufwand bei der Isolierung des Reaktionsproduktes durch mehrere Kondensationsstufen und einer nachgeschalteten Abgaswäsche.

Aus US-A-4,485,246 ist es außerdem bekannt, daß man Phthalid aus Phthalsäureanhydrid durch Hydrierung mit homogenen Rutheniumkatalysatoren herstellen kann. Bei diesen Verfahren ist die Katalysatorrückgewinnung schwierig.

In EP-A-542 037 wird weiter ein Verfahren zur Herstellung von Phthaliden durch katalytische Hydrierung von Phthalsäureanhydrid an Festbettkatalysatoren in Rohrreaktoren oder Rohrbündelreaktoren oder suspendierten Katalysatoren in einem Rührautoklaven beschrieben, das bei diskontinuierlicher Arbeitsweise hohe Aufwandmengen an Katalysator und lange Hydrierzeiten erfordert, während bei kontinuierlicher Arbeitsweise hohe Drücke erforderlich sind, um einen vollständigen Umsatz zu erreichen.

In EP-B-89 417 ist weiter die katalytische Hydrierung von Phthalsäureanhydrid zu Phthalid mit Hilfe eines auf einem Trägermaterial fixierten Nickelkatalysators unter Verwendung von Hubrührautoklaven beschrieben, wobei die Verwendung von Benzoesäuremethylester als Lösungsmittel zwingend vorgeschrieben ist. Das Verfahren hat den Nachteil hoher Aufwandmengen an Katalysator und langer Reaktionszeiten.

Aus Houben/Weyl, "Methoden der organischen Chemie", 6/2 (1963), Seiten 732 bis 733 sowie US-A-2,114,696 ist weiter die Hydrierung von Phthalsäureanhydrid zu Phthalid mit Hilfe von Raney-Nickel als Katalysator in einem Schüttelautoklaven bekannt, wobei bei einem Wasserstoffdruck von 165 bar in Ethanol als Lösungsmittel nur eine Ausbeute an Phthalid von 73 % erhalten wird.

Der vorliegenden Erfindung lag daher die Aufgabe zugrunde, den zuvor genannten Nachteilen der bekannten Verfahren bei der katalytischen Hydrierung von Phthalsäureanhydriden abzuhelfen. Demgemäß wurde ein neues und verbessertes Verfahren zur Herstellung von Phthaliden der allgemeinen Formel I, 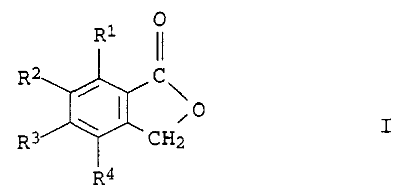 in der R¹, R², R³ und R⁴ unabhängig voneinander Wasserstoff, C₁-C₄-Alkyl oder C₁-C₄-Alkoxy bedeuten, durch Hydrierung von Phthalsäureanhydriden der allgemeinen Formel II, 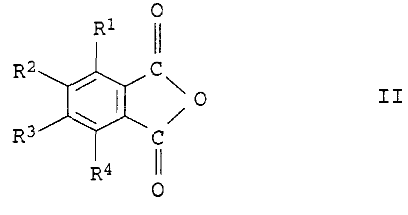 in der die Reste R¹, R², R³ und R⁴ die oben genannten Bedeutungen haben, mit Wasserstoff in Gegenwart von suspendierten Katalysatoren vom Raney-Typ in einer Hydriervorrichtung unter Vermischen einer den Katalysator und das eingesetzte Phthalsäureanhydrid und entstehendes Phthalid enthaltenden Flüssigphase und einer den Hydrierwasserstoff enthaltenden Gasphase mittels einer Mischvorrichtung gefunden, welches dadurch gekennzeichnet ist, daß mittels der Mischvorrichtung in die Flüssigphase Mischleistung von mindestens 50 W/l eingetragen wird.

Nach dem erfindungsgemäßen Verfahren läßt sich die Hydrierung von den Phthalsäureanhydriden zu den Phthaliden bei geringen Aufwandmengen der Katalysatoren mit guter Raum-Zeit-Ausbeute und hoher Selektivität durchführen.

Im Vergleich zu den herkömmlichen Verfahren zur Hydrierung von Phthalsäureanhydrid zu Phthalid mit Hilfe von suspendiertem Raney-Nickel erlaubt das erfindungsgemäße Verfahren die Aufwandmenge an Raney-Katalysatoren bei gleicher Hydrierzeit erheblich zu vermindern bzw. die Hydrierzeit bei vergleichbarer Aufwandmenge an Raney-Katalysatoren drastisch zu verkürzen.

Überraschenderweise wird, wie wir gefunden haben, bei dem erfindungsgemäßen Verfahren eine Desaktivierung der Raney-Katalysatoren, wie sie bei den herkömmlichen Verfahren unter Anwendung der üblichen Mischleistungen in Rühr- oder Schüttelautoklaven auftritt und die dort zu langen Reaktionszeiten und entsprechend geringen Raum-Zeit-Ausbeuten führt, vermieden. Außerdem werden die bei den herkömmlichen Verfahren auftretenden Verbackungen der Raney-Katalysatoren, die deren Abtrennung nach der Reaktion erschweren, bei dem erfindungsgemäßen Verfahren vermieden.

Wie wir weiter gefunden haben, dürfte die bei den herkömmlichen Verfahren auftretende Desaktivierung der Raney-Katalysatoren zurückzuführen sein auf die während der Hydrierung von Phthalsäureanhydrid zu Phthalid als störende Nebenprodukte entstehenden sauren Komponenten wie Toluylsäure und aus unumgesetztem Phthalsäureanhydrid und Reaktionswasser entstehende Phthalsäure, die mit den Raney-Metallen schwerlösliche Salze bilden, die sich wiederum vornehmlich auf der Katalysatoroberfläche abscheiden. Durch den beim erfindungsgemäßen Verfahren angewandten Eintrag sehr hoher Mischleistungen von mindestens 50 W/l werden durch die hierdurch bedingten hohen Scherkräfte Oberflächenbeläge schwerlöslicher Metallsalze wie Nickelsalze auf den suspendierten Raney-Katalysator-Teilchen ständig entfernt, so daß die Katalysatoroberfläche während der gesamten Reaktion aktiv bleibt (vgl. P.H.M.R. Cramers et al, Process Intensification with Buss Loop Reactors, in Proceedings of the Conference "Catalysis in Multiphase Reactors, Lyon, 7.-9.12.1994).

Es ist ein wesentliches Merkmal des erfindungsgemäßen Verfahrens, daß mittels der Mischvorrichtung, mit der bei der Hydrierung die Vermischung von Gas- und Flüssigphase erfolgt und gleichzeitig die Katalysatoren in suspendierter Form aufrechterhalten werden, in die Flüssigphase besonders hohe Mischleistungen und zwar von mindestens 50 W/l Flüssigphase, vorzugsweise 50 bis 2000 W/l Flüssigphase, insbesondere 100 bis 500 W/l Flüssigphase eingetragen werden.

Durch diese Arbeitsweise werden die vorstehend aufgeführten Vorteile gegenüber den herkömmlichen Hydrierverfahren mit Anwendung von üblichen Mischleistungen von 0,1 bis 10 W/l Flüssigphase, wie sie z.B. bei gerührten Reaktoren oder Schüttelautoklaven zur Anwendung kommen, erzielt.

Für die Durchführung des erfindungsgemäßen Verfahrens sind alle apparativen Anordnungen der Hydriervorrichtungen geeignet, die über entsprechende Mischvorrichtungen den Eintrag von mindestens 50 W/l ermöglichen.

Vorteilhaft wird das erfindungsgemäße Verfahren in einem Schleifenreaktor durchgeführt, bei dem mittels einer Umwälzpumpe die Flüssigphase aus dem Reaktor im Kreis geführt und am Kopf des Reaktors zurückgeführt wird. Die Zuführung kann beispielsweise in der Weise erfolgen, daß die rückgeführte flüssige Phase in feinverteilter Form in die gasförmige Wasserstoffatmosphäre des Reaktors gesprüht wird, wobei die erfindungsgemäße Mischleistung sowohl im Bereich der Umwälzpumpe als auch im Bereich der Sprühdüse auf die Flüssigphase übertragen werden kann. Mit besonderem Vorteil wird die erfindungsgemäße Hydrierung in einem Schleifenreaktor mit einem über eine Strahlsauger-Mischdüse gekoppelten Gas- und Flüssigkeits-Kreislauf durchgeführt. Beispielsweise wird das Verfahren mit Vorteil in einer Schleifenreaktor-Anlage, die folgende Merkmale aufweist, durchgeführt:
1: Reaktor
2: Umwälzpumpe
3: Wärmeaustauscher der Flüssigphase
4: Strahlsauger-Mischdüse
5: Umwälzleitung der Flüssigphase
6: Zufuhrleitung für Wasserstoff
7: Entleerungsleitung der Gasphase
8: Zufuhr- und Entleerungsleitung der Flüssigphase
9: gegebenenfalls geheizte Reaktorwandung
   a) Flüssigphase
   b) Gasphase.

Bei der Schleifenreaktor-Anlage werden geeignete hohe Mischungsleistungen insbesondere im Bereich der Umwälzpumpe 2, über die ein Leistungseintrag von bis zu ca. 100 W/l im Kreis geführte Flüssigphase erreicht werden kann, und im Bereich der Strahlsauger-Mischdüse 4, über die ein Leistungseintrag von 200 bis 800 W/l, stellenweise bis 10 000 W/l erreicht werden kann, eingetragen.

Die Ermittlung des Eintrags der Mischleistung mittels der Mischvorrichtung bzw. Mischvorrichtungen der Hydrierung erfolgt zweckmäßig in der in Ind. Eng. Chem. Res. **1992**, 31, Seiten 949 bis 958 beschriebenen Weise.

Die katalytische Hydrierung wird im allgemeinen bei Temperaturen von 50 bis 400°C, vorzugsweise 100 bis 250°C, insbesondere 140 bis 220°C und Drücken von 1 bis 400 bar, vorzugsweise 5 bis 300 bar, insbesondere 5 bis 200 bar, besonders vorteilhaft 30 bis 120 bar durchgeführt.

Als Hydrierkatalysatoren werden für das erfindungsgemäße Verfahren Raney-Katalysatoren verwendet. Geeignete Raney-Katalysatoren sind Raney-Nickel, Raney-Eisen, Raney-Kobalt, Raney-Kupfer, vorzugsweise Raney-Nickel.

Die Raney-Katalysatoren können zur Aktivitätssteigerung noch mit geeigneten Metallen dotiert werden. Beispielsweise kann es vorteilhaft sein, Raney-Nickel-Katalysatoren zu verwenden, die mit Metallen der 1. Nebengruppe des Periodensystems, im allgemeinen Silber, Kupfer, vorzugsweise Kupfer, mit Metallen der 6. Nebengruppe des Periodensystems, im allgemeinen Chrom, Molybdän oder Wolfram, vorzugsweise Molybdän und/oder mit Metallen der 7. Gruppe des Periodensystems, im allgemeinen Mangan, Rhenium, vorzugsweise Rhenium dotiert sind. Im allgemeinen beträgt der Gehalt der Raney-Nickel-Katalysatoren an den zur Dotierung zugesetzten Metallen 0,01 bis 10 Gew.-%, vorzugsweise 0,1 bis 5 Gew.-%, bezogen auf das in den Katalysatoren enthaltene Nickel.

Die erfindungsgemäß zu verwendenden Raney-Katalysatoren werden in üblicher Weise hergestellt. Beispielsweise erhält man Raney-Nickel, indem man Nickel und mit Lauge herauslösbare Stoffe, vorzugsweise Aluminium und/oder Silizium, legiert und die laugelöslichen Stoffe anschließend mit Lauge, wie Natronlauge herauslöst.

Die Hydrierung kann ohne Lösungsmittel durchgeführt werden, wobei das Phthalsäureanhydrid in geschmolzener Form eingesetzt wird.

In der Regel wird die Hydrierung unter Verwendung eines Lösungsmittels durchgeführt. Geeignete Lösungsmittel sind beispielsweise Ether wie Tetrahydrofuran, Dioxan, Glykolether, Ester wie Essigsäuremethylester und Methylbenzoat, Lactone wie Butyrolacton oder Phthalid, vorzugsweise das bei der Hydrierung gebildete Phthalid, Alkohole wie Methanol, Ethanol, Propanol, Butanol, Kohlenwasserstoffe oder Gemische dieser Lösungsmittel.

Das Gewichtsverhältnis von dem zu hydrierenden Phthalsäureanhydrid zu dem verwendeten Lösungsmittel beträgt im allgemeinen 1000:1 bis 1:1000, vorzugsweise 500:1 bis 1:500, insbesondere 200:1 bis 1:200.

Zweckmäßig wird man die erfindungsgemäße Umsetzung abbrechen, sobald alles eingesetzte Phthalsäureanhydrid hydriert ist. Der Zeitpunkt für den Abbruch wird z.B. durch Bestimmung des noch vorhandenen Phthalsäureanhydrids, beispielsweise durch Gas-Chromatographie oder aus dem zeitlichen Verlauf der Wasserstoffaufnahme ermittelt.

Die Aufarbeitung des Reaktionsproduktes erfolgt nach üblichen Methoden, vorzugsweise durch Destillation.

In den Verbindungen I und II bedeuten R¹, R², R³ und R⁴ unabhängig voneinander Wasserstoff; C₁-C₄-Alkyl wie Methyl, Ethyl, n-Propyl, iso-Propyl, n-Butyl, iso-Butyl, sec.-Butyl, tert.-Butyl, bevorzugt Methyl und Ethyl, insbesondere Methyl; C₁-C₄-Alkoxy wie Methoxy, Ethoxy, n-Propoxy, iso-Propoxy, n-Butoxy, iso-Butoxy, sec.-Butoxy, tert.-Butoxy, bevorzugt Methoxy und Ethoxy, insbesondere Methoxy. Besonders bevorzugt bedeuten alle Substituenten R¹, R², R³ und R⁴ Wasserstoff.

Die als Ausgangsstoffe verwendeten Phthalsäureanhydride II sind allgemein bekannt, wie das Phthalsäureanhydrid, oder können nach bekannten Verfahren erhalten werden (J. of Org. Chemistry 51, 3439-3446 (1986); Synthesis 223-224 (1985)).

Die Phthalide I eignen sich z.B. als Ausgangsstoff für die Synthese von Pflanzenschutzmitteln.

Die Erfindung wird durch folgende Beispiele verdeutlicht.

### Beispiel 1

In einem 70 l-Schleifenreaktor, werden 39,5 kg Phthalid als Lösungsmittel, 16,9 kg Phthalsäureanhydrid (PSA) und 169 g Raney-Nickel (B 113 W von Degussa), das in Butyrolacton suspendiert ist, bei 130°C als Schmelze vorgelegt. Der Reaktorinhalt wird mit 5 m³/h umgepumpt. Dabei wird in die Flüssigphase im Bereich der Umwälzpumpe Mischleistung von 100 W/l und im Bereich der Strahlsauger-Mischdüse Mischleistung von 1000 W/l eingetragen. Es wird Wasserstoff auf einen Druck von 40 bar aufgedrückt und der Reaktorinhalt innerhalb von 20 min auf 170°C erwärmt. Bei 40 bar und 170°C wird bis zur Druckkonstanz hydriert. Die Wasserstoffaufnahme beträgt 5700 Nl. Die Hydrierzeit beträgt 102 min.

Als Produkt wird eine farblose Schmelze erhalten der Zusammensetzung

| | |
|---|---|
| Phthalid | 94,1 Gew.-% |
| Wasser | 4,0 Gew.-% |
| PSA | 0,3 Gew.-% |
| Toluylsäure | 1,4 Gew.-% |

Dies entspricht einem Umsatz an PSA von 99 % bei einer Selektivität von 93 %.

### Beispiel 2 (Vergleichsbeispiel, nicht erfindungsgemäß)

In einem 0,5 l-Rührautoklav mit Begasungsrührer werden 88,8 g Phthalsäureanhydrid (PSA), 207,2 g Phthalid und 0,90 g Raney-Nikkel (B 113 W von Degussa) als Schmelze bei ca. 120°C vorgelegt. Nach Einschalten des Rührers wird Wasserstoff auf einen Druck von 40 bar aufgepreßt und der Reaktorinhalt auf 180°C erwärmt. Es wird bei diesem Druck und dieser Temperatur bis zur Druckkonstanz hydriert, wobei die Rührerdrehzahl 500 bzw. 1000 Upm beträgt. Die Versuchsbedingungen und die Ergebnisse der Hydrierung sind in der folgenden Tabelle zusammengefaßt.

| Versuchs- Nr. | Drehzahl | Hydrierzeit | Umsatz | Selektivität | Wasserstoffaufnahme |
|---|---|---|---|---|---|
| | Upm | min | % | % | Nl |
| 2a | 500 | 235 | 99,9 | 85 | 25,1 |
| 2b | 1000 | 225 | 99,7 | 83 | 24,8 |

Zusammensetzung der Produkte in Gew.-%

| Versuchs- Nr. | Phthalid | Wasser | PSA | Toluylsäure |
|---|---|---|---|---|
| 2a | 92,3 | 3,3 | <0,1 | 3,2 |
| 2b | 91,9 | 3,4 | 0,1 | 3,7 |

Im vorliegenden Vergleichsbeispiel sind gegenüber Beispiel 1 wesentlich längere Hydrierzeiten erforderlich, wobei überdies deutlich geringere Selektivitäten erzielt werden.

### Beispiel 3

Man verfährt wie in Beispiel 1 beschrieben, wobei jedoch die Reaktionstemperatur 182°C und der Druck 80 bar betragen.
PSA-Konzentration und Katalysatormenge werden gemäß folgender Tabelle variiert.

| Versuchs- Nr. | Konzentration von PSA bezogen auf PSA + Phthalid | Katalysatormenge | | Wasserstoffaufnahme | Hydrierzeit | Umsatz | Selektivität |
|---|---|---|---|---|---|---|---|
| | Gew.-% | g | Gew.-%, bezogen auf PSA | N1 | min | % | % |
| 3a | 30 | 43 | 0,25¹⁾ | 5800 | 160 | 99 | 88 |
| 3b | 30 | 54 | 0,35²⁾ | 5200 | 144 | 99 | 88,5 |
| 3c | 50 | 210 | 0,75²⁾ | 9700 | 65 | 100 | 88,6 |

| | | | | | | | |
|---|---|---|---|---|---|---|---|
| ¹⁾ Butyrolacton-feucht | | | | | | | |
| ²⁾ Wasser-feucht | | | | | | | |

### Beispiel 4 (Vergleichsbeispiel, nicht erfindungsgemäß)

In einem 2 l-Autoklav mit Hubrührer werden 413 g Phthalsäureanhydrid (PSA), 964 g Phthalid (für 4c 688,5 g PSA und 688,5 g Phthalid) und die in der Tabelle angegebene Menge Raney-Nickel als Schmelze vorgelegt. Nach Inertisieren mit Stickstoff und Austausch gegen Wasserstoff wird bei einer Hubzahl von 160 je Minute auf die angegebene Temperatur aufgeheizt und der angegebene Wasserstoffdruck aufgepreßt. Es wird unter diesen Bedingungen bis zur Druckkonstanz hydriert.

Die Versuchsbedingungen und Ergebnisse sind in der folgenden Tabelle zusammengefaßt:

| Versuchs- Nr. | Temperatur | Druck | Katalysatormenge | | Wasserstoffaufnahme | Hydrierzeit | Umsatz | Selektivität |
|---|---|---|---|---|---|---|---|---|
| | °C | bar | g | Gew.-%, bezogen auf PSA | N1 | min | % | % |
| 4a | 170 | 40 | 4,13 | 1,0¹⁾ | 136 | 365 | 100 | 75 |
| 4b | 183 | 80 | 1,03 | 0,25¹⁾ | 137 | 1162 | 100 | 79 |
| 4c | 183 | 80 | 1,45 | 0,35¹⁾ | 137 | 435 | 100 | 85 |
| 4d | 183 | 80 | 5,16 | 0,75²⁾ | 208 | 164 | 99 | 87 |

| | | | | | | | | |
|---|---|---|---|---|---|---|---|---|
| ¹⁾ Butyrolacton-feucht | | | | | | | | |
| ²⁾ Wasser-feucht | | | | | | | | |

Im vorliegenden Vergleichsbeispiel sind gegenüber Beispiel 3 wesentlich längere Hydrierzeiten erforderlich.

## Patentansprüche

1. Verfahren zur Herstellung von Phthaliden der allgemeinen Formel I, 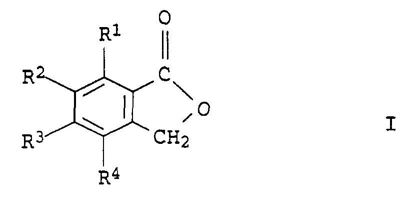 in der R¹, R², R³ und R⁴ unabhängig voneinander Wasserstoff, C₁-C₄-Alkyl oder C₁-C₄-Alkoxy bedeuten, durch Hydrierung von Phthalsäureanhydriden der allgemeinen Formel II, 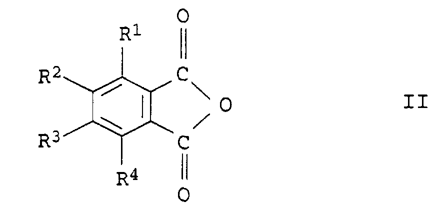 in der die Reste R¹, R², R³ und R⁴ die oben genannten Bedeutungen haben, mit Wasserstoff in Gegenwart von suspendierten Katalysatoren vom Raney-Typ in einer Hydriervorrichtung unter Vermischen einer den Katalysator und das eingesetzte Phthalsäureanhydrid und entstehendes Phthalid enthaltenden Flüssigphase und einer den Hydrierwasserstoff enthaltenden Gasphase mittels einer Mischvorrichtung, dadurch gekennzeichnet, daß mittels der Mischvorrichtung in die Flüssigphase Mischleistung von mindestens 50 W/l eingetragen wird.

2. Verfahren nach Anspruch 1, dadurch gekennzeichnet, daß der Eintrag der Mischleistung 50 bis 10 000 W/l beträgt.

3. Verfahren nach Anspruch 1 und 2, dadurch gekennzeichnet, daß man die Hydrierung in einem Schleifenreaktor mit über eine Strahlsauger-Mischdüse gekoppeltem Gas- und Flüssigkeits-Kreislauf durchführt.

4. Verfahren nach den Ansprüchen 1 bis 3, dadurch gekennzeichnet, daß als Raney-Katalysatoren Raney-Nickel, Raney-Kobalt oder Raney-Kupfer verwendet werden.

5. Verfahren nach Anspruch 4, dadurch gekennzeichnet, daß als Raney-Katalysator Raney-Nickel verwendet wird.

## Claims

1. A process for preparing phthalides of the general formula I, 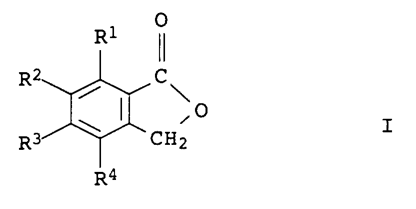 where R¹, R², R³ and R⁴ are independently hydrogen, C₁-C₄-alkyl or C₁-C₄-alkoxy, by hydrogenating phthalic anhydrides of the general formula II, 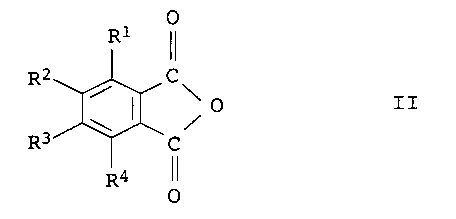 where R¹, R², R³ and R⁴ are each as defined above, with hydrogen in the presence of suspended catalysts of the Raney type in a hydrogenation apparatus by using a mixing apparatus to mix a liquid phase, which includes the catalyst and the phthalic anhydride used and resulting phthalide, and a gas phase, which includes the hydrogenating hydrogen, which comprises using the mixing apparatus to introduce a mixing intensity of at least 50 W/l into the liquid phase.

2. A process as claimed in claim 1, wherein the mixing intensity introduced is within the range from 50 to 10,000 W/l.

3. A process as claimed in claim 1 or 2, wherein the hydrogenation is carried out in a loop reactor comprising a gas and liquid recirculation system coupled via a multistream ejector mixing nozzle.

4. A process as claimed in any of claims 1 to 3, wherein the Raney catalysts used are Raney nickel, Raney cobalt or Raney copper.

5. A process as claimed in claim 4, wherein the Raney catalyst used is Raney nickel.

## Revendications

1. Procédé pour la préparation de phtalides de formule générale I 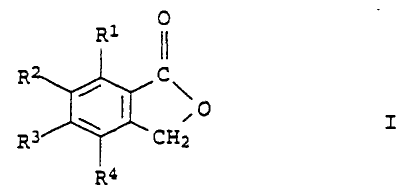 dans laquelle R¹, R², R³ et R⁴ représentent chacun, indépendamment les uns des autres, l'hydrogène, un groupe alkyle en C1-C4 ou alcoxy en C1-C4, par hydrogénation des anhydrides phtaliques de formule générale II 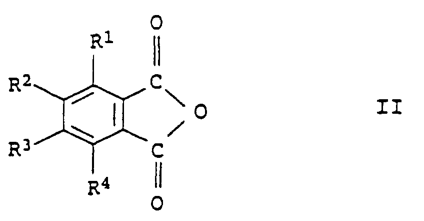 dans laquelle les symboles R¹, R², R³ et R⁴ ont les significations indiquées ci-dessus, en présence de catalyseurs en suspension du type Raney dans un appareil d'hydrogénation avec mélange d'une phase liquide contenant le catalyseur, l'anhydride phtalique mis en oeuvre et le phtalide formé et d'une phase gazeuse contenant l'hydrogène à l'aide d'un appareil de mélange, caractérisé par le fait que l'appareil de mélange exerce sur la phase liquide une puissance de mélange d'au moins 50 W/l.

2. Procédé selon la revendication 1, caractérisé par le fait que la puissance de mélange exercée est de 50 à 10 000 W/l.

3. Procédé selon les revendications 1 et 2, caractérisé par le fait que l'on procède à l'hydrogénation dans un réacteur à boucle avec un circuit de gaz et un circuit de liquide couplés par une tuyère de mélange aspiratrice à jet.

4. Procédé selon les revendications 1 à 3, caractérisé par le fait que l'on utilise en tant que catalyseurs de Raney, le nickel de Raney, le cobalt de Raney ou le cuivre de Raney.

5. Procédé selon la revendication 4, caractérisé par le fait que le catalyseur de Raney est du nickel de Raney.
